# EUROPEAN PATENT APPLICATION

(11) **EP 0 808 626 A1**
(43) Date of publication of application: **26.11.1997**
(21) Application number: 96830532.6
(22) Date of filing: 16.10.1996
(51) Int. Cl.: A61K 31/22

(54) **Use of acetyl-L-carnitine for the treatment of presenile Alzheimer's disease**

(30) Priority: 24.05.1996 IT RM960360
(71) Applicant: Sigma-Tau Industrie Farmaceutiche Riunite S.p.A., 00144 Roma (IT)
(72) Inventor: Calvani, Menotti, 00146 Rome (IT); Carta, Angelico, 00144 Rome (IT)
(74) Representative: Fassi, Aldo, Dr.

(57) **Abstract**

The use of acetyl L-carnitine and the pharmacologically acceptable salts thereof is disclosed for producing a medicament selectively suitable for slowing down the worsening of the symptoms in patients affected from the presenile form of Alzheimer 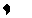 s disease (AD) ( 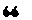 early-onset 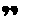 AD patients).

## Description

The present invention relates to the use of acetyl L-carnitine and its pharmacologically acceptable salts to produce a medicament for the treatment of Alzheimer's disease (AD).

More specifically, the present invention relates to the use of the aforesaid compounds to produce a medicament to slow down the worsening of the symptoms typical of this disease selectively in patients suffering from the presenile form of AD, or early-onset AD patients, wherein the expression 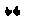 early-onset AD 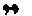 shall be subsequently rigorously set forth pursuant to the definition laid down by the American Psychiatric Association.

Alzheimer's disease is a progressive, irreversible degenerative process of the brain which expresses itself clinically through impairment of the intellectual functions, the first and most manifest symptom being loss of short-term memory and subsequently of the cognitive functions. With progression of the disease, long-term memory is also distinctly impaired and the patient proves to be increasingly disoriented in terms first of temporal relationships, and then of spatial relationships, and, in the end, in terms of his or her relationships with people. The patient's ability to formulate judgements and abstract thoughts deteriorates rapidly, while alterations of mood become increasingly evident. In the final stage of the disease the patient is bed-ridden and incontinent, loses all contact with the real world, and his or her senses and intellectual faculties deteriorate totally.

Since similar cerebral alterations (neuritic plaques, beta-amyloid plaques and neuro-fibrillary degenerations) are detected by the pathologist at autopsy both in relatively young patients with early-onset AD and in elderly patients with late-onset dementia, it was long believed that there were no substantial significant differences between these two groups of patients.

However, definitively convincing clinical, genetic and neurobiological data have now been acquired and conclusive evidence has been achieved demonstrating that patients with early onset of Alzheimer's disease (referred to hereinafter for short as early-onset AD patients) constitute a population which is quite distinct from the population of patients with late onset of the disease (referred to hereinafter for short as late-onset AD patients) so much so as to suggest that differentiated therapeutic approaches should be contemplated for the early- versus late-onset AD populations. Among the abundant literature on the subject reference is made, for example, to the very recent publication by E. Koss *et al.* "Clinical and neurophysiological differences between patients with earlier and later onset of Alzheimer's disease: A. CERAD analysis, part XII in Neurology, 46, 136-141 (1996).

A further finding which has now been definitely established is that early-onset patients tend to deteriorate more rapidly than late-onset patients.

Though to date a diagnosis of definite Alzheimer's disease still requires anatomico-pathological confirmation, considerable progress has been made over the past 15 years in identifying and establishing clinical criteria for the diagnosis of this disease. The identification of these criteria is of particular importance for drawing up accurate research protocols and for establishing reproducible recruitment practices for patients to be included in controlled clinical trials, which is a *sine qua non* requirement for significant comparison of the various clinical trials.

The study of these criteria has been jointly conducted in the USA by the National Institute of Neurological and Communicative Disorders and Stroke (NINCDS) and by the Alzheimer's Disease and Related Disorders Association (ADRDA). These criteria are compatible with the definitions provided by the Diagnostic and Statistical Manual of Mental Disorders (DSM IV R) published by the American Psychiatric Association. In this connection reference is made to the publication by G. McKhann *et al.* "Clinical diagnosis of Alzheimer's disease; Report of the NINCDS-ADRDA Work Group under the auspices of the Department of Health and Human Services Task Force on Alzheimer's Disease" in Neurology, 34, 939-944 (1984).

In the latest edition of the Diagnostic and Statistical Manual of Mental Disorders (DSM IV R) the distinction between the early-onset subgroup (defined as the subpopulation in which the dementia sets in at an age of 65 years or less) and the late-onset subgroup (subpopulation in which the age of onset is above 65 years) was fully acknowledged (cf. DSM IV R pp. 140-143), with the result that it no longer appears possible to dispense with this distinction as an inclusion criterion in controlled clinical trials aimed at assessing the efficacy of candidate drugs for the treatment of Alzheimer's disease.

The only drug licensed in the USA and currently marketed for the treatment of Alzheimer's disease is TACRINE (tetrahydroaminoacridine), a cholinesterase inhibitor about whose efficacy many doubts have been raised, whereas there is no doubt about its potent liver toxicity. For instance, Martindale's authoritative Extra Pharmacopoeia, 30th edition (1993) states:
*"The cholinesterase inhibitor tacrine ... is the subject of much controversy. One of the original studies in which it was reported to produce encouraging improvements in some symptoms of senile dementia was much criticised and on subsequent investigation the FDA concluded that flaws in the conduct of the study damaged the credibility of the results .... . Later studies which were considered to demonstrate little benefit have also been criticised".*

Moreover, Goodman and Gilman's "The Pharmalogical Basis of Therapeutics", 9th edition (1996), reports:
*"While tacrine represents the only current therapeutic option for the treatment of Alzheimer's disease, it should be recognized that most suitable patient candidates will not benefit substantially from the treatment regimens. Dosing regimens, weekly monitoring of alanine aminotransferase, and the limitations of cholinergic side effects require substantive oversight on the part of the physician".*

Lastly, Drug Information 1995, published by the American Hospital Formulary Service, commenting on the results of a 30-month clinical study with tacrine, concludes:
*"These findings and those from several other studies have been less favorable than those reported by an earlier, controversial study of the drug, and there currently is no evidence from well-designed studies that therapy with the drug can alter the underlying disease process of dementia".*

In the past few years, on the basis of the teachings in US patent 4.346.107 which discloses and claims a therapeutic method for the treatment of patients suffering from altered cerebral metabolism, Alzheimer's disease and other diseases of the central nervous system which comprises administering, as an active ingredient, an acyl derivative of carnitine, which may be acetyl carnitine, various clinical trials have been conducted aimed at assessing the efficacy of acetyl L-carnitine in slowing down the progression of Alzheimer's disease in patients suffering from the mild-to-moderate forms of the disease.

The results of these studies have proved strongly contradictory, producing no appreciable degree of certainty as to the efficacy of acetyl L-carnitine.

For example, Spagnoli *et al.,* in Neurology, 41, 1726-1732 (1991), describe the results of a double-blind study in which a cohort of 130 patients with Alzheimer's disease were treated with acetyl L-carnitine for 1 year.

At the end of the treatment, both the group treated with acetyl L-carnitine and the one treated with placebo had deteriorated, but the group treated with acetyl L-carnitine showed a slower rate of deterioration in 13 out of 14 preselected primary parameters (or endpoints), reaching statistical significance for the Blessed Dementia Scale, logical intelligence, ideomotor and buccofacial apraxia, and selective attention.

By contrast, a recent study conducted in the United States in a substantially larger population (431 patients, 83% of whom completed the scheduled treatment year) with the cognitive component of the Alzheimer's Disease Assessment Scale (ADAS-Cog) and the Clinical Dementia Rating Scale constituting the main endpoints, failed to confirm the results of the above-mentioned study by Spagnoli *et al.* In fact, at the end of the US study both the patients treated with acetyl L-carnitine and those treated with placebo were found to have deteriorated at the same rate as regards the main endpoints and most of the secondary endpoints.

It is essential to note that, in both the above-mentioned studies, the patients treated constituted an undifferentiated population in terms of age of onset of dementia and that the statistical interpretation of the study was done on the entire patient cohort without any distinction between early- and late-onset subpopulations.

It has now been found, as a conclusion of a retrospective analysis of a series of double-blind, placebo-controlled trials, which will be described below in greater detail, that selective treatment with acetyl L-carnitine of patients defined as early-onset on the basis of the criterion laid down in the Diagnostic and Statistical Manual of Mental Disorders, 4th edition, revised (DSM-IV-R) achieves a statistically significant slower decline of the selected primary endpoints, particularly ADAS-Cog and CDR, in such patients as compared to early-onset AD patients treated with placebo.

This favourable therapeutic result would be totally obscured if the group treated with acetyl L-carnitine also included a significantly large number of late-onset AD patients and the statistical interpretation of the study were extended to both subpopulations considered as a single cohort.

If, as previously mentioned, we consider that early-onset AD patients tend to deteriorate more rapidly than late-onset patients and that there is no theoretical reason why an active ingredient which is effective in slowing down a disease in a group of patients with a tendency to deteriorate more rapidly should not be more effective in slowing down the disease in a group of patients with a lesser tendency to deteriorate, the selective efficacy of acetyl L-carnitine in the treatment of early-onset AD patients proves unexpected and surprising.

Therefore, the present invention relates to the use of acetyl L-carnitine or its pharmacologically acceptable salts to produce a medicament suitable for slowing down the deterioration of the symptom picture in patients with early-onset Alzheimer's disease.

More particularly, the present invention relates to the use of acetyl L-carnitine or its pharmacologically acceptable salts to produce a medicament suitable for selectively slowing down the deterioration of the symptom picture in those patients who fulfil the definition of early-onset AD patients according to the standard practices of the Diagnostic and Statistical Manual of Mental Disorders, 4th edition, revised (DSM-IV-R) of the American Psychiatric Association.

It has also been found that, although the daily dose to be administered depends, according to the judgement of the primary care physician, on the subject's weight, age and general condition, it is generally advisable to administer from 1 to 4 g/day, and preferably 2 to 3 g/day of acetyl L-carnitine or an equimolar amount of one of its pharmacologically acceptable salts, even if larger doses can be administered in view of the substantial non-toxicity of acetyl L-carnitine.

The medicament of the invention can be obtained by mixing the active ingredient (acetyl L-carnitine or one of its pharmaceutically acceptable salts) with appropriate excipients suitable for the formulation of compositions which lend themselves to enteral administration (particularly oral) or to parenteral administration (particularly intramuscular or intravenous). All such excipients are well known to experts in pharmacy and pharmaceutical technology.

What is meant by pharmacologically acceptable salts of acetyl L-carnitine are any of its salts with an acid that does not give rise to unwanted side effects. Such acids are well known to pharmacologists and to experts in pharmacy and pharmaceutical technology.

Non-exclusive examples of such salts are chloride, bromide, orotate, acid aspartate, citric acid, acid phosphate, fumarate and acid fumarate, lactate, maleate and acid maleate, acid oxalate, acid sulphate, glucose phosphate, tartrate and acid tartrate.

A number of examples of formulations in the form of unitary doses are provided here below.
**(a) Formulation for tablets**
   One tablet contains:
   **Active ingredient**
      - acetyl L-carnitine.HCl 590 mg
         (corresponding to 500 mg of acetyl L-carnitine, inner salt)
   **Excipients**
      - microcrystalline cellulose. polyvinylpyrrolidone, magnesium stearate, cellulose
         acetate phthalate, diethylphthalate, dimethicone
**(b) Formulation for intravenous injectable ampoules**
   One ampoule contains:
   **Active ingredient**
      - acetyl L-carnitine 500 mg
   **Excipients**
      - mannitol
         One solvent ampoule contains:
      - water for injections, q.s. up to 5 ml
**(C) Formulation for sachets**
   One sachet contains:
   **Active ingredient**
      - acetyl L-carnitine.HCl 590 mg
         (corresponding to 500 mg of acetyl L-carnitine, inner salt)
   **Excipients**
      - silica gel, sodium saccharine, hydroxypropyl-cellulose sodium bicarbonate,
         tonic water (1 x 1000), mannitol.
**(d) Formulation for extemporaneous solution**
   One 12.316 g vial contains:
   **Active ingredient**
      - acetyl L-carnitine.HCl 12.0 g
         (corresponding to 10.17 g base)
   **Excipients**
      - methyl p-hydroxybenzoate, propyl p-hydroxybenzoate polyvinylpyrrolidone.

There now follows a brief description of a clinical study demonstrating the selective efficacy of acetyl L-carnitine in slowing down the deterioration of primary parameters in a group of early-onset AD patients.

### CLINICAL STUDY

For the purposes of assessing the clinical efficacy of acetyl L-carnitine in early-onset AD patients. 8 different parallel-group, double-blind, randomized, placebo-controlled trials in which acetyl L-carnitine was administered chronically by mouth to subjects suffering from early-onset (before age 65) or late-onset (after age 65) Alzheimer's disease were pooled and assessed as a whole.

Of these 8 trials 2 were multicentre studies (one conducted in the USA and the other in Italy) with a duration of 44 weeks, while the other 5 (1 in the USA, 3 in the UK, and 1 in Italy) had a duration of 6 months. Acetyl L-carnitine or placebo were administered at doses ranging from 2 to 3 g/day in the different studies. All patients recruited into these trials had diagnoses of probable Alzheimer's disease according to the criteria laid down by the DSM-III-R and the NINCDS-ADRDA; for recruitment patients had to have a Hachinski Ischemic Score of 4 points or less and no CT or NMR findings indicative of cerebral lesions other than those typical of Alzheimer's disease.

A total of 834 patients were recruited into the 8 trials considered. Of these, 140 (16.8%) were suffering from early-onset AD (before 65 years of age according to the DSM-IV definition). The criterion adopted to identity these patients as early-onset was their actual age at the time of recruitment into the study.

A statistical meta-analysis was then used to retrospectively assess the progressive course of Alzheimer's disease in these 140 subjects, who proved to be well matched numerically in their randomization to the group treated with acetyl L-carnitine (n = 68) and to that treated with placebo (n = 72) in the 8 trials (Table 1). Two hypotheses were tested:
a) that the early-onset AD group on placebo presented a more rapid progression of AD compared to the late-onset patients on placebo; and
b) that the early-onset group treated with acetyl L-carnitine showed a slower progression of the disease compared to the early-onset patients receiving placebo.

In the 8 trials considered, the progression of Alzheimer's disease was assessed on the basis of cognitive clinical rating scales (cognitive section of the the Alzheimer's Disease Assessment Scale [ADAS-Cog]; MMSE - Mini Mental State Examination; BIMC - Blessed Information Memory Concentration) and functional or global clinical scales (CDR - Clinical Dementia Rating Scale; BDS - Blessed Dementia Scale; CGI-S and -C - Clinical Global Impression of Severity and Change) according to the scientific criteria indicated and approved by the various international regulatory authorities.

The statistical analysis, in this case defined as a meta-analysis for the retrospective and global evaluation of different studies, was accomplished by applying a non-parametric analysis standardized for ranks according to an extension of the Mantel-Haenszel method. The use of standardized ranks for each efficacy measure, whether cognitive or functional, made it possible to pool homogeneous information from different studies, assessing the differences between the baseline and end-of-study examinations for the so-called "efficacy" population or, at any rate, the last post-baseline examination carried out for the "intention-to-treat" population. For the purposes of standardizing the different durations of the studies analyzed, a mean end-of-treatment value was calculated, ranging from 6 to 12 months according to the duration of each study. Again following the Mantel-Haenszel method, the application of logranks was also evaluated instead of the standardized ranks so as to better highlight any particular group of responders.

### RESULTS

The descriptive analysis of the clinical trend of the placebo-treated early-onset AD patients compared to the late-onset patients shows, both for single studies and overall, that the early-onset AD patients present more rapid progression of the disease, thus confirming the results of other clinical trials and conferring reliability upon the comparison between acetyl L-carnitine and placebo in the early-onset patient group.

The clinical characteristics of the early-onset AD patients at the baseline examination proved to be well matched between subjects randomly assigned to treatment with acetyl L-carnitine and those assigned to treatment with placebo in the 8 trials (Tables 2 and 3). The meta-analysis of the cognitive and functional efficacy measures clearly shows a homogeneous tendency, as emerging from the various clinical rating scales utilized, on the part of early-onset AD patients treated with acetyl L-carnitine to present a slower progression of the disease compared to the early-onset patients on placebo (Table 4), the difference reaching statistical significance in both the "intention-to-treat" and the "efficacy" populations in numerous cognitive and functional assessments, as shown in Tables 5 and 6.

**TABLE 1**

| Essential elements of the study. | | | | | |
|---|---|---|---|---|---|
| Study code | Country | Duration | Age (min, max) | Number of patients (male percentage) | Doses (grams) |
| ST-901 | U.S. | 12 months | 60 (51,65) | 83 (41%) | 3 |
| ST-88102 | U.S. | 44 weeks | 62 (60,65) | 10 (90%) | 2-3 |
| DRN2-006 | Italy | 12 months | 61 (56,65) | 16 (33%) | 2 |
| ST-88101 | U.S. | 6 months | 62 (58,64) | 11 (27%) | 2.5-3 |
| S104CT1 | U.K. | 6 months | 65 (65,65) | 2 (0%) | 2 |
| S104CT03 | U.K. | 6 months | 64 (62,65) | 3 (33%) | 2 |
| S104CT04 | U.K. | 6 months | 63 (61,65) | 2 (0%) | 2 |
| SR-32189 | Italy | 6 months | 59 (55,64) | 15 (53%) | 2 |

**TABLE 2**

| Descriptive statistics for cognitive parameters at baseline for intention-to-treat population. | | | | | | | |
|---|---|---|---|---|---|---|---|
| Study code | Parameter | Acetyl L-carnitine | | | Placebo | | |
| | | N | Mean | Range | N | Mean | Range |
| ST-901 | ADAS-Cog | 36 | 28 | (12, 47) | 47 | 26 | (9, 49) |
| | MMSE | 36 | 19 | (12, 25) | 46 | 20 | (13, 26) |
| ST-88102 | ADAS-Cog | 6 | 18 | (8, 34) | 4 | 18 | (9, 29) |
| | MMSE | 6 | 19 | (12, 24) | 4 | 22 | (18, 28) |
| DRN2-006 | BIMC | 8 | 17 | (10, 23) | 7 | 17 | (14, 20) |
| 6 months* | MMSE | 18 | 20 | (14, 24) | 14 | 18 | (14, 23) |
| ADAS-Cog = cognitive section of Alzheimer's Disease Assessment Scale MMSE = Mini Mental State Examination BIMC = Blessed Information Memory Concentration 6 months* refers to the 5 studies in which AD patients were treated double-blind for 6 months | | | | | | | |

**TABLE 3**

| Descriptive statistics for functional parameters at baseline for intention-to-treat population. | | | | | | | |
|---|---|---|---|---|---|---|---|
| Study code | Parameter | Acetyl L-carnitine | | | Placebo | | |
| | | N | Mean | Range | N | Mean | Range |
| ST-901 | CDR | 34 | 6.1 | (3, 12) | 44 | 6.3 | (1.5, 13) |
| | CGI-S | 36 | 3.5 | (3.5) | 47 | 3.5 | (2, 5) |
| ST-88102 | BDS | 6 | 6.0 | (3.5, 12.5) | 4 | 5.3 | (2, 11.5) |
| | CGI-S | 6 | 4.0 | (3.5) | 4 | 3.8 | (3, 4) |
| DRN2-006 | BDS | 8 | 12.9 | (7, 17) | 7 | 9.8 | (2.5, 16.5) |
| 6 months* | CGI-S | 17 | 3.5 | (3.5) | 14 | 3.1 | (2, 4) |
| CDR = Clinical Dementia Rating Scale CGI-S = Clinical Global Impression - Severity BDS = Blessed Dementia Scale | | | | | | | |

**TABLE 4**

| Descriptive statistics for mean changes in final values at 6-12 months of cognitive and functional parameters "efficacy" population compared to baseline | | | | | | | | |
|---|---|---|---|---|---|---|---|---|
| Study code | Type of parameter | Parameter | Acetyl L-carnitine | | | Placebo | | |
| | | | N | Mean | S.E. | N | Mean | S.E. |
| ST-901 | Cognitive | ADAS-Cog | 28 | 28 | 1.8 | 40 | 5.8 | 0.9 |
| | | MMSE | 32 | -27 | 0.8 | 40 | -4.0 | 0.5 |
| ST-88102 | Cognitive | ADAS-Cog | 6 | 4.7 | 1.4 | 4 | 3.5 | 3.9 |
| | | MMSE | 6 | -2.1 | 1.1 | 4 | -1.8 | 1.1 |
| DRN2-006 | Cognitive | BIMC | 7 | -3.5 | 3.1 | 7 | -6.3 | 1.2 |
| 6 months | Cognitive | MMSE | 17 | -0.7 | 0.7 | 14 | -1.9 | 0.8 |
| ST-901 | Functional | CDR | 32 | 1.9 | 0.5 | 40 | 2.9 | 0.4 |
| | | CGI-S | 32 | 0.3 | 0.1 | 40 | 0.3 | 0.1 |
| | | CGI-C | 32 | 4.5 | 0.1 | 40 | 4.7 | 0.1 |
| ST-88102 | Functional | BDS | 6 | 1.2 | 0.6 | 4 | 1.5 | 2.1 |
| | | CGI-S | 6 | 0.0 | 0.0 | 4 | 0.2 | 0.2 |
| | | CGI-C | 6 | 4.4 | 0.2 | 4 | 4.0 | 0.8 |
| DRN2-006 | Functional | BDS | 7 | 0.4 | 1.1 | 7 | 4.0 | 1.6 |
| 6 months | Functional | CGI-S | 17 | 0.2 | 0.1 | 14 | 0.5 | 0.2 |
| | | CGI-C | 17 | 4.3 | 0.3 | 14 | 4.6 | 0.3 |

**TABLE 5**

| *P* values for pooled studies comparing treatments in terms of differences between final and baseline values. | | | | | | | |
|---|---|---|---|---|---|---|---|
| Type of parameter | Study components | | | | Score | Efficacy *P* value | I-T-T* *P* value |
| | ST-901 | ST-88102 | DRN2-006 | 6 months | | | |
| Cognitive | ADAS-Cog | ADAS-Cog | BIMC | MMSE | Rank | 0.104 | 0.170 |
| Cognitive | ADAS-Cog | ADAS-Cog | BIMC | MMSE | Logrank | 0.045 | 0.114 |
| Cognitive | MMSE | MMSE | BIMC | MMSE | Rank | 0.269 | 0.218 |
| Cognitive | MMSE | MMSE | BIMC | MMSE | Logrank | 0.103 | 0.099 |
| Functional | CDR | CGI-S | BDS | CGI-S | Rank | 0.025 | 0.006 |
| Functional | CDR | CGI-S | BDS | CGI-S | Logrank | 0.006 | 0.002 |
| Functional | CGI-S | CGI-S | BDS | CGI-S | Rank | 0.071 | 0.028 |
| Functional | CGI-S | CGI-S | BDS | CGI-S | Logrank | 0.058 | 0.025 |
| Functional | CGI-C | CGI-C | BDS | CGI-C | Rank | 0.149 | 0.159 |
| Functional | CGI-C | CGI-C | BDS | CGI-C | Logrank | 0.223 | 0.243 |

| | | | | | | | |
|---|---|---|---|---|---|---|---|
| *I-T-T = Intention-to-treat | | | | | | | |

**TABLE 6**

| *P* values for pooled studies comparing treatments in terms of differences between mean final and base-line values. | | | | | | | |
|---|---|---|---|---|---|---|---|
| Type of parameter | Study components | | | | Score | Efficacy *P* value | I-T-T* *P* value |
| | ST-901 | ST-88102 | DRN2-006 | 6 months | | | |
| Cognitive | ADAS-Cog | ADAS-Cog | BIMC | MMSE | Rank | 0.074 | 0.155 |
| Cognitive | ADAS-Cog | ADAS-Cog | BIMC | MMSE | Logrank | 0.035 | 0.074 |
| Cognitive | MMSE | MMSE | BIMC | MMSE | Rank | 0.049 | 0.044 |
| Cognitive | MMSE | MMSE | BIMC | MMSE | Logrank | 0.019 | 0.022 |
| Functional | CDR | CGI-S | BDS | CGI-S | Rank | 0.015 | 0.004 |
| Functional | CDR | CGI-S | BDS | CGI-S | Logrank | 0.004 | 0.001 |
| Functional | CGI-S | CGI-S | BDS | CGI-S | Rank | 0.066 | 0.037 |
| Functional | CGI-S | CGI-S | BDS | CGI-S | Logrank | 0.064 | 0.037 |
| Functional | CGI-C | CGI-C | BDS | CGI-C | Rank | 0.233 | 0.311 |
| Functional | CGI-C | CGI-C | BDS | CGI-C | Logrank | 0.440 | 0.545 |

| | | | | | | | |
|---|---|---|---|---|---|---|---|
| *I-T-T = Intention-to-treat | | | | | | | |

## Claims

1. Use of acetyl L-carnitine and the pharmacologically acceptable salts thereof for producing a medicament suitable for slowing down the worsening of the symptoms of Alzheimer's disease (AD) in patients suffering from the presenile form of AD.

2. The use of claim 1, characterized in that the medicament is selectively suited for the treatment of patients who comply with the definition of 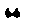 early-onset AD patients 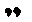 pursuant to the criteria laid down in the Diagnostic and Statistical Manual of Mental Disorders, 4th ed., revised (DSM-IV-R) published by the American Psychiatric Association.

3. The use of claim 2, characterized in that the medicament is selectively suitable for slowing down the worsening of the primary endpoints in early-onset AD patients.

4. The use of claim 2, characterized in that the medicament is selectively suitable for slowing down the worsening of ADAS-Cog and CDR in early-onset AD patients.

5. The use of claims 1-4 wherein the medicament is orally administrable.

6. The use of claim 1-4 wherein the medicament is parenterally administrable.

7. The use of claim 6 wherein the medicament is intravenously administered.

8. The use of any of the preceding claims wherein the pharmacologically acceptable salt of acetyl L-carnitine is selected from chloride, bromide, orotate, acid aspartate citric acid, acid phosphate, fumarate and acid fumarate, lactate, maleate and acid maleate, acid oxalate, acid sulphate, glucose phosphate, tartrate and acid tartrate.
